# EUROPEAN PATENT APPLICATION

(11) **EP 3 355 050 A1**
(43) Date of publication of application: **01.08.2018**
(21) Application number: 17789059.7
(22) Date of filing: 28.02.2017
(51) Int. Cl.: G01N 27/00, G01N 27/06, G01N 27/22

(54) **SENSOR**

(30) Priority: 27.04.2016 JP 2016089097
(71) Applicant: KYB Corporation, Tokyo 105-6111 (JP)
(72) Inventor: YOSHIDA, Takahiro, Tokyo 105-6111 (JP); KAMEDA, Yukinori, Tokyo 105-6111 (JP)
(74) Representative: Horn Kleimann Waitzhofer Patentanwälte PartG mbB
(86) International application number: PCT/JP2017/007846
(87) International publication number: WO 2017/187770

(57) **Abstract**

A sensor is provided, configured to provide further detailed judgment with respect to the state of a fluid. A sensor (1) configured to judge the state of a fluid used in a device comprises a characteristics acquisition unit (10), a time measurement unit (20), and a state judgment unit (30). The characteristics acquisition unit (10) acquires the dielectric constant and the conductivity of the fluid. The time measurement unit (20) measures the time that elapses from the time point at which the fluid was charged to the device. The state judgment unit (30) judges the state of the fluid based on the dielectric constant and the conductivity acquired by the characteristics acquisition unit (10) and the time measured by the time measurement unit (20).

## Description

### Technical Field

The present invention relates to a sensor.

### Background Art

A technique for judging a state of a fluid has been proposed (see Patent document 1, for example).

An oil degradation detection apparatus is described in Patent document 1, configured to judge whether or not degradation has occurred in oil that is supplied to a rotating member or a sliding member in a circulating manner so as to allow such a member to operate smoothly while suppressing abrasion. The oil degradation detection apparatus includes a pair of electrode plates arranged in parallel with each other along an oil flow path. By applying an AC voltage across the pair of electrode plates, the oil degradation detection apparatus acquires the conductivity and the dielectric constant of the oil, and judges whether or not oil degradation has occurred based the conductivity and the dielectric constant thus acquired.

### Citation List

### Patent Literature

[Patent Document 1] Japanese Patent Application Laid Open No. 2009-2693

### Summary of Invention

### Technical Problem

The oil degradation apparatus described in Patent document 1 judges whether or not oil degradation has occurred, and classifies the oil state into four states, i.e., a normal state, an abnormal state due to increased soot contamination, an abnormal state due to increased water contamination, and an abnormal state due to thermal degradation.

By obtaining further detailed information with respect to the state of the fluid, this allows maintenance to be supported more appropriately. Accordingly, there is a demand for obtaining further detailed information with respect to the state of the fluid.

Accordingly, the present invention has been made in order to solve the aforementioned problem. It is a purpose of the present invention to provide a sensor configured to judge the state of a fluid in a more detailed manner.

### Solution of Problem

At least one embodiment of the present invention relates to a sensor that judges a state of a fluid in a device. The sensor comprises: a characteristics acquisition unit that acquires a dielectric constant and a conductivity of the fluid; a time measurement unit that measures a time period from a timing at which the fluid was charged to the device; and a state judgment unit that judges the state of the fluid based on the dielectric constant and the conductivity acquired by the characteristics acquisition unit and the time period measured by the time measurement unit.

### Brief Description of the Drawings

Fig. 1 is a block diagram showing a sensor according to an embodiment of the present invention.
Fig. 2 is a schematic configuration diagram showing a characteristics acquisition unit included in the sensor according to an embodiment of the present invention.
Fig. 3 is a graph showing the changes in the dielectric constant and the conductivity of the lubricant oil over time when the lubricant oil is in the state ST1.
Fig. 4 is a graph showing the changes in the dielectric constant and the conductivity of the lubricant oil over time when the lubricant oil is in the state ST2.
Fig. 5 is a graph showing the changes in the dielectric constant and the conductivity of the lubricant oil over time when the lubricant oil is in the state ST3.
Fig. 6 is a graph showing the changes in the dielectric constant and the conductivity of the lubricant oil over time when the lubricant oil is in the state ST4.
Fig. 7 is a graph showing the changes in the dielectric constant and the conductivity of the lubricant oil over time when the lubricant oil is in the state ST4.
Fig. 8 is a graph showing the changes in the dielectric constant and the conductivity of the lubricant oil over time when the lubricant oil is in the state ST4.
Fig. 9 is a graph showing the changes in the dielectric constant and the conductivity of the lubricant oil over time when a lubricant oil is in the state ST5.
Fig. 10 is a graph showing the changes in the dielectric constant and the conductivity of the lubricant oil over time when the lubricant oil is in the state ST6.
Fig. 11 is a graph showing the changes in the dielectric constant and the conductivity of the lubricant oil over time when the lubricant oil is in the state ST7.
Fig. 12 is a graph showing the changes in the dielectric constant and the conductivity of the lubricant oil over time when the lubricant oil is in the state ST8.
Fig. 13 is a graph showing the changes in the dielectric constant and the conductivity of the lubricant oil over time when the lubricant oil is in the state ST9.
Fig. 14 is a flowchart showing an operation of the sensor according to an embodiment of the present invention.
Fig. 15 is a flowchart showing an operation of the sensor according to an embodiment of the present invention.
Fig. 16 is a flowchart showing an operation of the sensor according to an embodiment of the present invention.
Fig. 17 is a flowchart showing an operation of the sensor according to an embodiment of the present invention.
Fig. 18 is a flowchart showing an operation of the sensor according to an embodiment of the present invention.

### Description of Embodiments

Description will be made below regarding an embodiment of the present invention with reference to the drawings. It should be noted that a component or element of the following embodiment may be substituted by a different existing component or element as appropriate. Also, various kinds of variations may be made including a combination with a different existing component or element. Thus, description of the following embodiment is by no means intended to restrict the scope of the invention described in the appended claims.

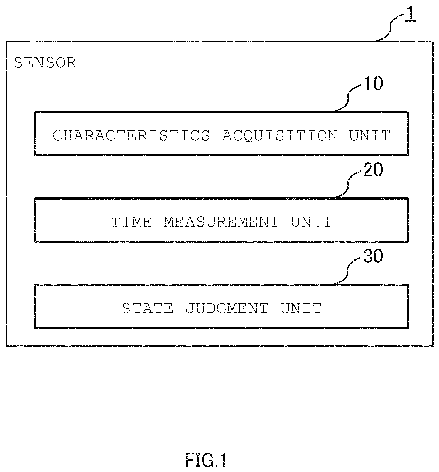

Fig. 1 is a block diagram showing a sensor 1 according to an embodiment of the present invention. The sensor 1 includes a characteristics acquisition unit 10, a time measurement unit 20, and a state judgment unit 30, and judges the state of lubricant oil used in a device. The characteristics acquisition unit 10 acquires the dielectric constant and the conductivity of the lubricant oil. The time measurement unit 20 measures the time that elapses from a time point at which the lubricant oil is charged to the device. The state judgment unit 30 judges the state of the lubricant oil based on the dielectric constant and the conductivity acquired by the characteristics acquisition unit 10 and the time measured by the time measurement unit 20.

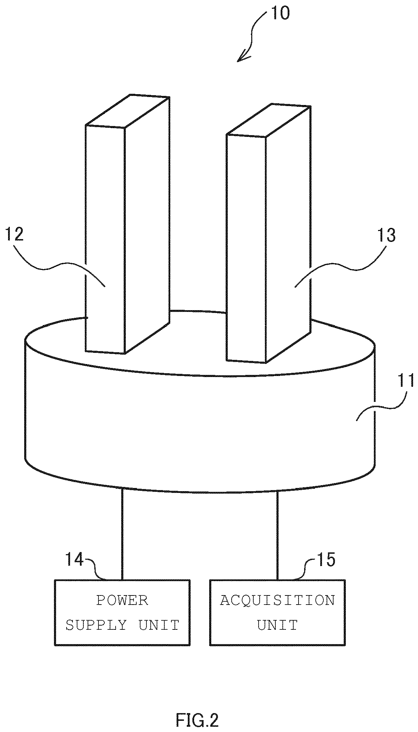

Fig. 2 is a schematic configuration diagram showing the characteristics acquisition unit 10. The characteristics acquisition unit 10 includes a circular base 11, a pair of electrodes 12 and 13 arranged such that they stand erect on the base 11, a power supply unit 14, and an acquisition unit 15.

The electrodes 12 and 13 are each configured as a planar-shaped electrode plate, and are arranged in parallel with each other. The power supply unit 14 applies an AC voltage across the electrodes 12 and 13 arranged in the lubricant oil. The acquisition unit 15 acquires the dielectric constant and the conductivity of the lubricant oil interposed between the electrodes 12 and 13 in a state in which the AC voltage is applied across the electrodes 12 and 13 by means of the power supply unit 14.

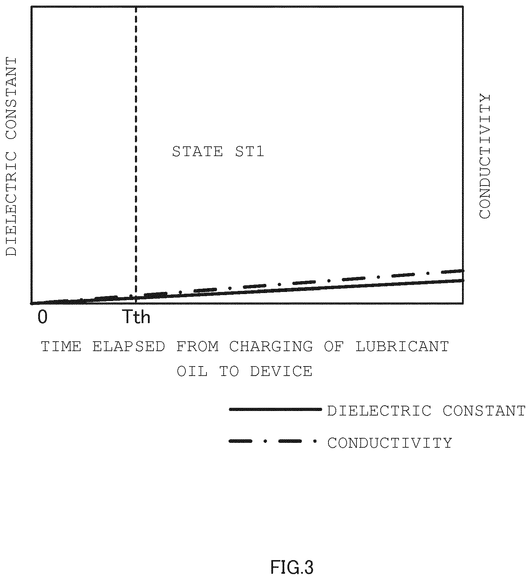

It should be noted that, in the present embodiment, the state judgment unit 30 judges the state of the lubricant oil from among nine states, i.e., states ST1 through ST9. The nine states will be described below with reference to Figs. 3 through 13. It should be noted that, in Figs. 3 through 13, the horizontal axis represents the time from the time point at which the lubricant oil is charged to the device. Furthermore, the first vertical axis represents the dielectric constant of the lubricant oil in the device. The second vertical axis represents the conductivity of the lubricant oil in the device.

### [State ST1]

Fig. 3 is a graph showing changes in the dielectric constant and the conductivity of the lubricant oil over time in a case in which the lubricant oil charged to the device degrades normally. In this case, the change in the dielectric constant per unit of time (dielectric constant slope) Δε and the change in the conductivity per unit of time (conductivity slope) Δσ remain relatively small. In this case, the state judgment unit 30 judges that the lubricant oil is degrading normally. That is to say, the state judgment unit 30 judges that the lubricant oil is in the state ST1.

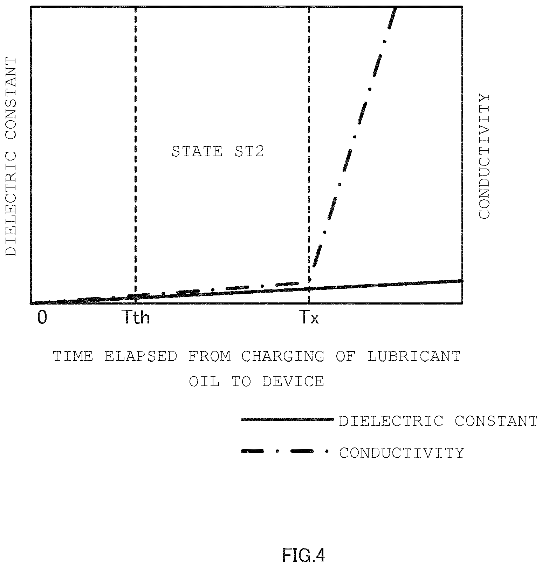

It should be noted that, in the present embodiment, such a case in which the lubricant agent is degrading normally represents a case in which the lubricant oil is degrading naturally. In other words, this represents a case in which the lubricant oil is degrading without any one of the abnormal states described later with reference to Figs. 4 through 13.

### [State ST2]

Fig. 4 is a graph showing changes in the dielectric constant and the conductivity of the lubricant oil over time in a case in which foreign material contamination occurs in the lubricant oil or otherwise abnormal abrasion occurs in the device due to damage in the device or the like after the lubricant oil was charged to the device. In this case, the change in the dielectric constant per unit of time Δε remains relatively small regardless of the passage of time. In contrast, the change in the conductivity per unit of time Δσ remains relatively small regardless of the passage of time before the time point Tx. Here, the time point Tx represents a time point at which such foreign material contamination or abnormal abrasion occurs. After the time point Tx, the change in the conductivity per unit of time Δσ becomes relatively large as compared with that before the time point Tx. In this case, the state judgment unit 30 judges that foreign material contamination or abnormal abrasion has occurred. That is to say, the state judgment unit 30 judges that the lubricant oil is in the state ST2.

### [State ST3]

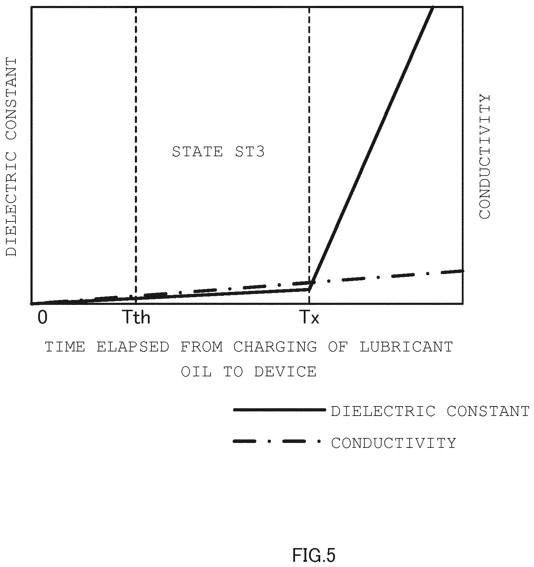

Fig. 5 is a graph showing the changes in the dielectric constant and the conductivity of the lubricant oil over time in a case in which sudden oxidation degradation has occurred in the lubricant oil due to depletion of an additive such as an antioxidant or due to thermal degradation after the lubricant oil was charged to the device. In this case, the change in the conductivity per unit of time Δσ remains relatively small regardless of the passage of time. In contrast, the change in the dielectric constant per unit of time Δε remains relatively small regardless of the passage of time before the time point Tx. Here, the time point Tx represents a time point at which such sudden oxidation degradation has started. After the time point Tx, the change in the dielectric constant per unit of time Δε becomes relatively large as compared with that before the time point Tx. In this case, the state judgment unit 30 judges that sudden oxidation degradation has occurred after the lubricant oil is charged to the device. That is to say, the state judgment unit 30 judges that the lubricant oil is in the state ST3.

### [State ST4]

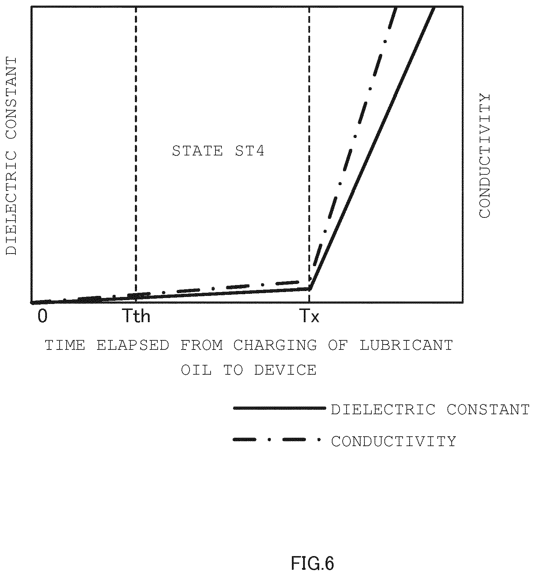

Fig. 6 is a graph showing the changes in the dielectric constant and the conductivity of the lubricant oil over time in a case in which the lubricant oil has been contaminated with coolant, water, or the like, due to the occurrence of a malfunction in the device after the lubricant oil was charged to the device. In this case, the change in the dielectric constant per unit of time Δε and the change in the conductivity per unit of time Δσ remain relatively small regardless of the passage of time before the time point Tx. Here, the time point Tx represents a time point at which the lubricant oil has been contaminated with coolant, water, or the like. After the time point Tx, the change in the dielectric constant per unit of time Δε and the change in the conductivity per unit of time Δσ become relatively large as compared with those before the time point Tx. In this case, the state judgment unit 30 judges that the lubricant oil has been contaminated with coolant, water, or the like after the lubricant oil was charged to the device. That is to say, the state judgment unit 30 judges that the lubricant oil is in the state ST4.

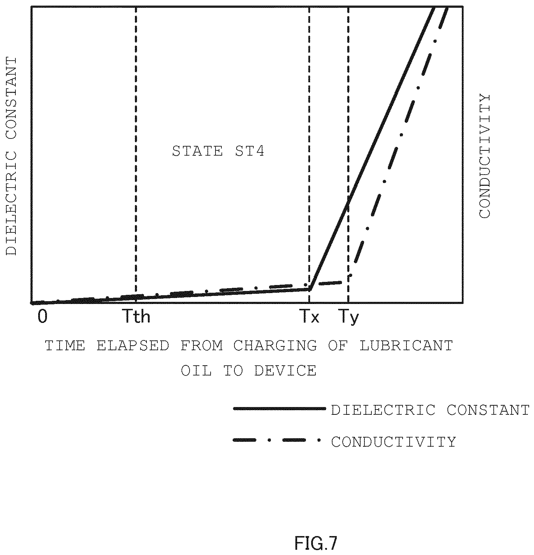

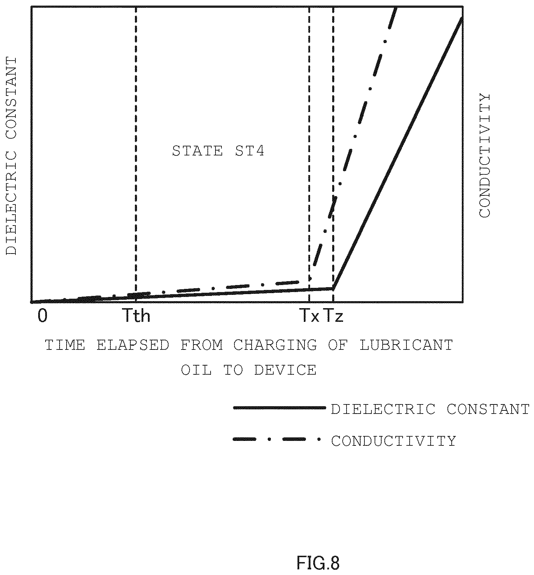

It should be noted that, in Fig. 6, the change in the dielectric constant per unit of time Δε and the change in the conductivity per unit of time Δσ change at the time point Tx, i.e., at the same timing. However, as shown in Fig. 7, such an arrangement has the potential to involve a state in which the change in the dielectric constant per unit of time Δε changes at the time point Tx, following which the change in the conductivity per unit of time Δσ changes at the time point Ty. Also, as shown in Fig. 8, such an arrangement has the potential to involve a state in which the change in the conductivity per unit of time Δσ changes at the time point Tx, following which the change in the dielectric constant per unit of time Δε changes at the time point Tz. In such cases shown in Figs. 7 and 8, the state judgment unit 30 judges that the lubricant oil has been contaminated with coolant, water, or the like after the lubricant oil was charged to the device. That is to say, the state judgment unit 30 judges that the lubricant oil is in the state ST4.

It should be noted that the time from the time point Tx to the time point Ty and the time from the time point Tx to the time point Tz are designed as appropriate according to the kind of the lubricant oil, the kind of the device, the operation state of the device, etc.

### [State ST5]

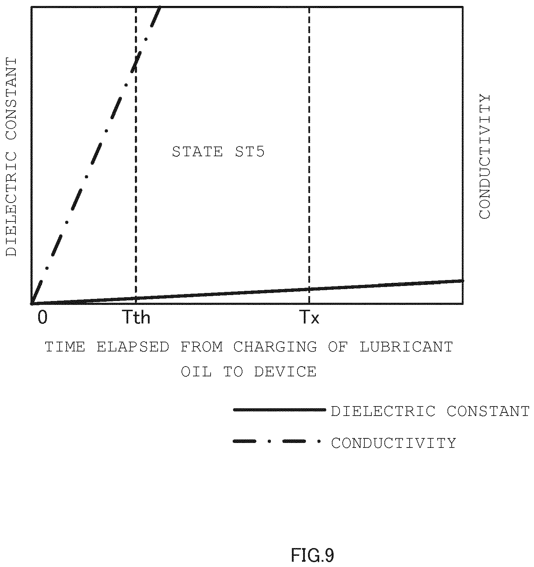

Fig. 9 is a graph showing the changes in the dielectric constant and the conductivity of the lubricant oil over time in a case in which foreign material contamination has occurred in the lubricant oil or otherwise abnormal abrasion has occurred in the device due to damage in the device when or otherwise before the lubricant oil is charged to the device. In this case, the change in the dielectric constant per unit of time Δε remains relatively small regardless of the passage of time. In contrast, the change in the conductivity per unit of time Δσ becomes large immediately after the lubricant oil is charged to the device. In this case, the state judgment unit 30 judges that foreign material contamination has occurred in the lubricant oil or otherwise abnormal abrasion has occurred in the device when or otherwise before the lubricant oil is charged to the device. That is to say, the state judgment unit 30 judges that the lubricant oil is in the state ST5.

### [State ST6]

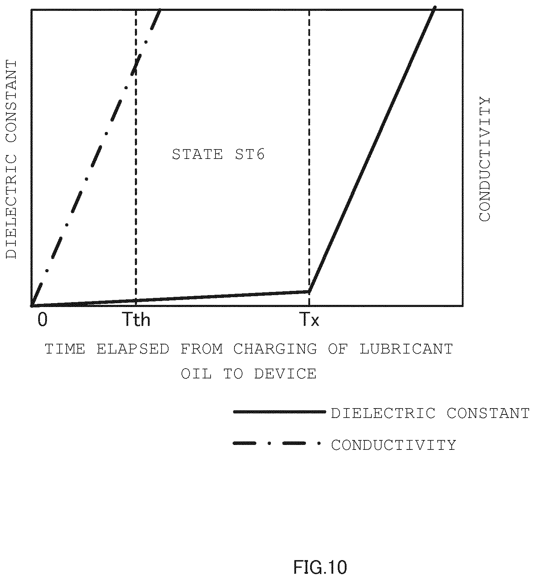

Fig. 10 is a graph showing the changes in the dielectric constant and the conductivity of the lubricant oil over time in a case in which foreign material contamination has occurred in the lubricant oil or otherwise abnormal abrasion has occurred in the device due to damage in the device when or otherwise before the lubricant oil is charged to the device, and in a case in which sudden oxidation degradation has occurred in the lubricant oil due to depletion of an additive such as an antioxidant or due to thermal degradation after the lubricant oil is charged to the device. In this case, the change in the conductivity per unit of time Δσ becomes large immediately after the lubricant oil is charged to the device. In contrast, the change in the dielectric constant per unit of time Δε remains relatively small regardless of the passage of time before the time point Tx. Here, the time point Tx represents a time point at which such sudden oxidation degradation has started. After the time point Tx, the change in the dielectric constant per unit of time Δε becomes relatively large as compared with that before the time point Tx. In this case, the state judgment unit 30 judges that foreign material contamination has occurred in the lubricant oil or otherwise abnormal abrasion has occurred in the device when or otherwise before the lubricant oil is charged to the device, and that sudden oxidation degradation has occurred after the lubricant oil is charged to the device. That is to say, the state judgment unit 30 judges that the lubricant oil is in the state ST6.

### [State ST7]

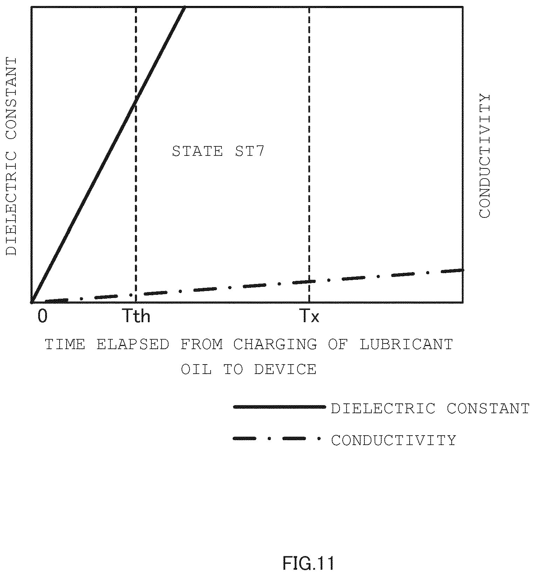

Fig. 11 is a graph showing the changes in the dielectric constant and the conductivity of the lubricant oil over time in a case in which the device is required to be subjected to flushing immediately after the lubricant oil is charged to the device. In this case, the change in the conductivity per unit of time Δσ remains relatively small regardless of the passage of time. In contrast, the change in the dielectric constant per unit of time Δε becomes large immediately after the lubricant oil is charged to the device. In this case, the state judgment unit 30 judges that the device is required to be subjected to flushing immediately after the lubricant oil is charged to the device. That is to say, the state judgment unit 30 judges that the lubricant oil is in the state ST7.

It should be noted that such a state in which the device is required to be subjected to flushing represents a state in which there is a great amount of sludge or the like in the device, for example.

### [State ST8]

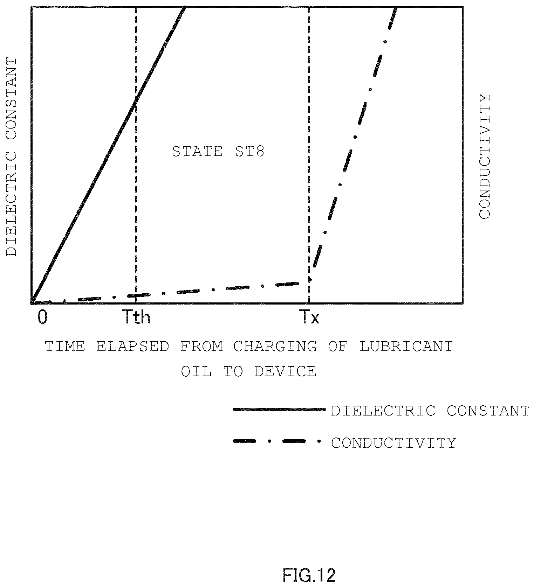

Fig. 12 is a graph showing the changes in the dielectric constant and the conductivity of the lubricant oil over time in a case in which the device is required to be subjected to flushing before or otherwise when the lubricant oil is charged to the device, and in a case in which foreign material contamination occurs in the lubricant oil or otherwise abnormal abrasion occurs after the lubricant oil is charged to the device. In this case, the change in the dielectric constant per unit of time Δε becomes large immediately after the lubricant oil is charged to the device. In contrast, the change in the conductivity per unit of time Δσ remains relatively small regardless of the passage of time before the time point Tx. Here, the time point Tx represents a time point at which such foreign material contamination or abnormal abrasion has occurred. After the time point Tx, the change in the conductivity per unit of time Δσ becomes large as compared with that before the time point Tx. In this case, the state judgment unit 30 judges that the device is required to be subjected to flushing before or otherwise when the lubricant oil is charged to the device, and that foreign material contamination or abnormal abrasion has occurred after the lubricant oil is charged to the device. That is to say, the state judgment unit 30 judges that the lubricant oil is in the state ST8.

### [State ST9]

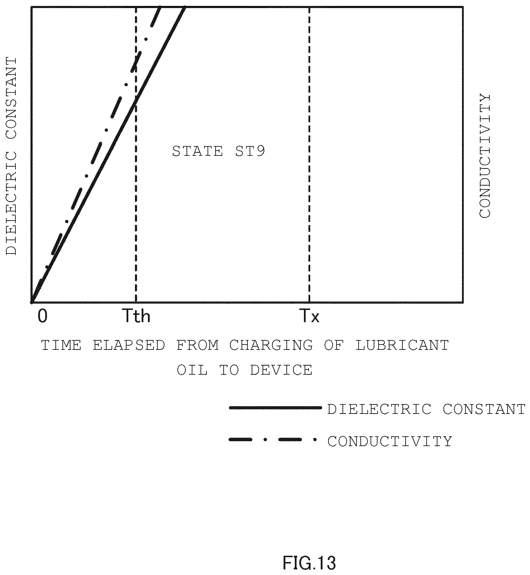

Fig. 13 is a graph showing the changes in the dielectric constant and the conductivity of the lubricant oil over time in a case in which the device is required to be subjected to flushing and the lubricant oil has been contaminated with coolant, water, or the like before or otherwise when the lubricant oil is charged to the device. In this case, the change in the dielectric constant per unit of time Δε and the change in the conductivity per unit of time Δσ become large immediately after the lubricant oil is charged to the device. In this case, the state judgment unit 30 judges that the device is required to be subjected to flushing and the lubricant oil has been contaminated with coolant, water, or the like, before or otherwise when the lubricant oil is charged to the device. That is to say, the state judgment unit 30 judges that the lubricant oil is in the state ST9.

The characteristics acquisition unit 10, the time measurement unit 20, and the state judgement unit 30, described above, are provided using a CPU, memory (RAM), hard disk, etc.

The hard disk stores an operating system, a program for executing a sequence of operations for judging the state of the lubricant oil, etc. It should be noted that the hard disk may be configured as a non-temporary recording medium. For example, instead of such a hard disk, nonvolatile memory such as EPROM or flash memory, CD-ROM, or the like may be employed.

The CPU reads out the data and programs stored in the hard disk as appropriate, using the memory as appropriate, and executes calculation and processing as appropriate.

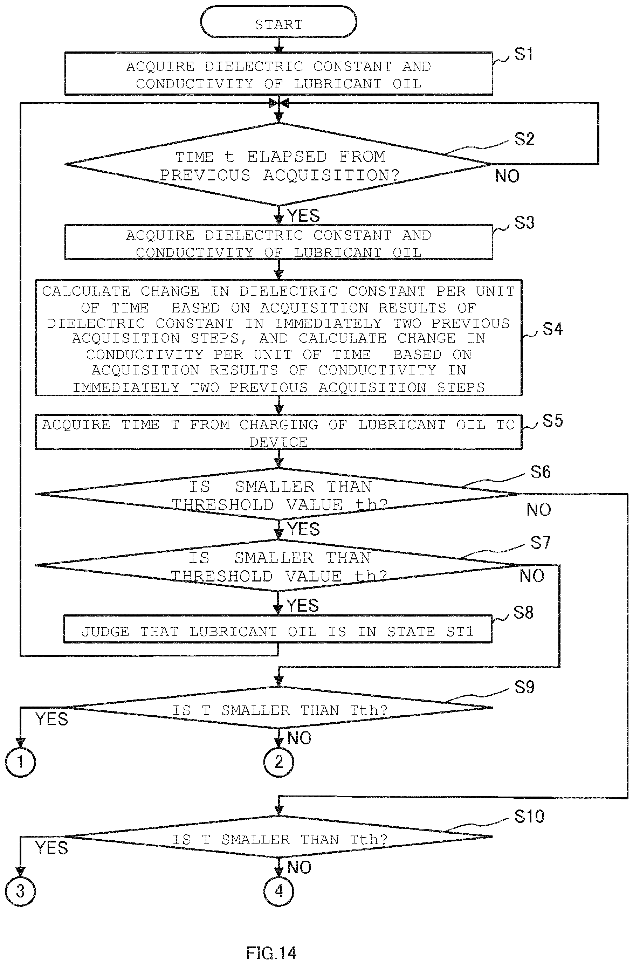

Figs. 14 through 18 are flowcharts each showing the operations of the sensor 1. The operations shown in Figs. 14 through 18 are repeatedly executed from Step S1 every time a predetermined period of time elapses.

In Step S1, the sensor 1 instructs the characteristics acquisition unit 10 to acquire the dielectric constant and the conductivity of the lubricant oil used in the device, following which the flow transits to Step 2.

In Step S2, the sensor 1 judges by means of the time measurement unit 20 whether or not a predetermined sampling time Δt or more has elapsed from the timing at which the characteristics acquisition unit 10 acquired the dielectric constant and the conductivity in the previous characteristics acquisition step. When the sensor 1 has judged by means of the time measurement unit 20 that the predetermined sampling time Δt or more has elapsed, the flow transits to Step S3. Conversely, when the sensor 1 has judged that the predetermined sampling time Δt has not elapsed, the operation in Step S2 is repeatedly performed.

It should be noted that the sampling time Δt is a period of time required to twice acquire each of the dielectric constant and the conductivity, and to calculate the change in the dielectric constant per unit of time Δε and the change in the conductivity per unit of time Δσ based on the results thus acquired. Accordingly, the sampling time Δt is designed as appropriate according to the kind of the lubricant oil, the kind of the apparatus, the operation state of the apparatus, etc.

In Step S3, the sensor 1 instructs the characteristics acquisition unit 10 to acquire the dielectric constant and the conductivity of the lubricant oil used in the device, following which the flow transits to Step S4.

In Step S4, the sensor 1 instructs the state judgment unit 30 to calculate the change in the dielectric constant per unit of time Δε based on measurement results of the dielectric constant acquired in two immediately previous executions of the acquisition step by means of the characteristics acquisition unit 10. Furthermore, the sensor 1 instructs the state judgment unit 30 to calculate the change in the conductivity per unit of time Δσ based on measurement results of the conductivity acquired in two immediately previous executions of the acquisition step by means of the characteristics acquisition unit 10. Subsequently, the flow transits to Step S5.

In Step S5, the sensor 1 instructs the time measurement unit 20 to acquire the time T that elapses from the time point at which the lubricant oil is charged, following which the operation transits to Step S6.

In Step S6, the sensor 1 instructs the state judgment unit 30 to judge whether or not the change in the dielectric constant per unit of time Δε is smaller than a threshold value Δεth. When the sensor 1 has judged by means of the state judgment unit 30 that the change in the dielectric constant per unit of time Δε is smaller than the threshold value Δεth, the sensor 1 switches the operation to Step S7. Conversely, when the sensor 1 has judged that the change in the dielectric constant per unit of time Δε is equal to or greater than the threshold value Δεth, the sensor 1 switches the operation to Step S10.

It should be noted that, as described above with reference to Figs. 3 through 13, the threshold value Δεth is a value defined so as to judge whether or not the change in the dielectric constant per unit of time Δε is small or otherwise large. Accordingly, the threshold value Δεth is designed as appropriate according to the kind of the lubricant oil, the kind of the device, the operation state of the device, etc.

In Step S7, the sensor 1 instructs the state judgment unit 30 to judge whether or not the change in the conductivity per unit of time Δσ is smaller than a threshold value Δσth. When the sensor 1 has judged by means of the state judgment unit 30 that the change in the conductivity per unit of time Δσ is smaller than the threshold value Δσth, the sensor 1 switches the operation to Step S8. Conversely, when the sensor 1 has judged that the change in the conductivity per unit of time Δσ is equal to or greater than the threshold value Δσth, the sensor 1 switches the operation to Step S9.

It should be noted that, as described above with reference to Figs. 3 through 13, the threshold value Δσth is a value defined so as to judge whether or not the change in the conductivity per unit of time Δσ is small or otherwise large. Accordingly, the threshold value Δσth is designed as appropriate according to the kind of the lubricant oil, the kind of the device, the operation state of the device, etc.

In Step S8, the sensor 1 judges by means of the state judgement unit 30 that the lubricant oil in the device is in the state ST1, following which the operation is returned to Step S2.

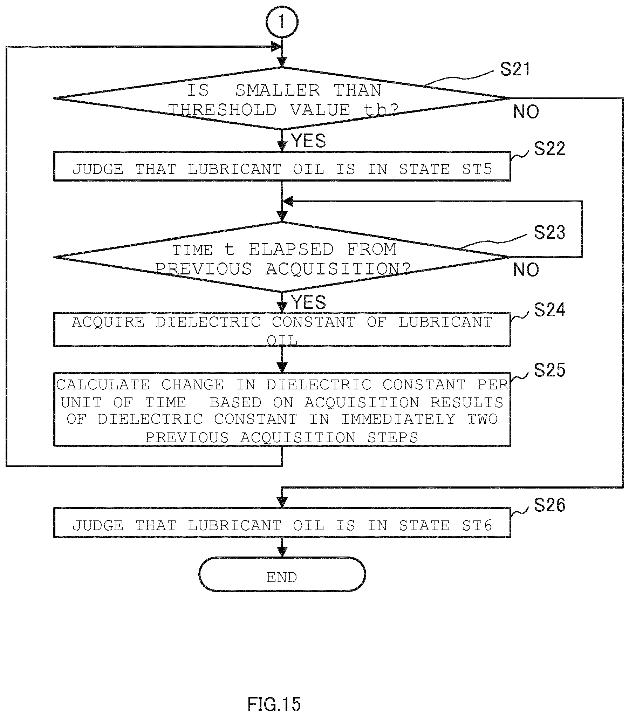

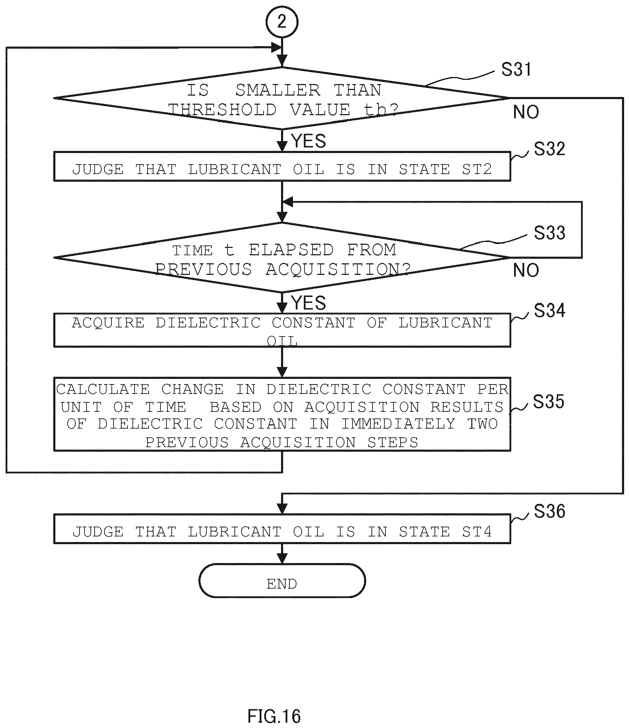

In Step S9, the sensor 1 instructs the state judgment unit 30 to judge whether or not the time T acquired in the Step S5 is smaller than a threshold value Tth. When the sensor 1 has judged by means of the state judgment unit 30 that the time T is smaller than the threshold value Tth, the sensor 1 switches the operation to Step 21 shown in Fig. 15. Conversely, when the sensor 1 has judged that the time T is equal to or greater than the threshold value Tth, the sensor 1 switches the operation to Step S31 shown in Fig. 16.

It should be noted that the timing at which an abnormal state has occurred in the lubricant oil used in the device can be classified into two cases, as described above with reference to Figs. 4 thorough 13.

One is a case in which an abnormal state has occurred in the device when or otherwise before the lubricant oil is charged to the device. In this case, an abnormal state occurs in the lubricant oil immediately after the lubricant oil is charged to the device.

Another is a case in which an abnormal state has occurred in the device or otherwise the degradation level of the lubricant oil advances and exceeds a predetermined level after the lubricant oil has been charged to the device. In this case, an abnormal state does not occur in the lubricant oil immediately after the lubricant oil is charged to the device. However, an abnormal state occurs in the lubricant oil at a time point at which an abnormal state has occurred in the device or otherwise a time point at which the degradation level of the lubricant oil advances and exceeds a predetermined level.

The threshold value Tth is a value defined in order to judge whether or not such an abnormal state described above with reference to Figs. 4 through 13 has occurred when or before the lubricant oil is charged to the device or otherwise such an abnormal state has occurred after the lubricant oil is charged to the device. The threshold value Tth is designed as appropriate according to the kind of the lubricant oil, the kind of the device, the operation state of the device, etc.

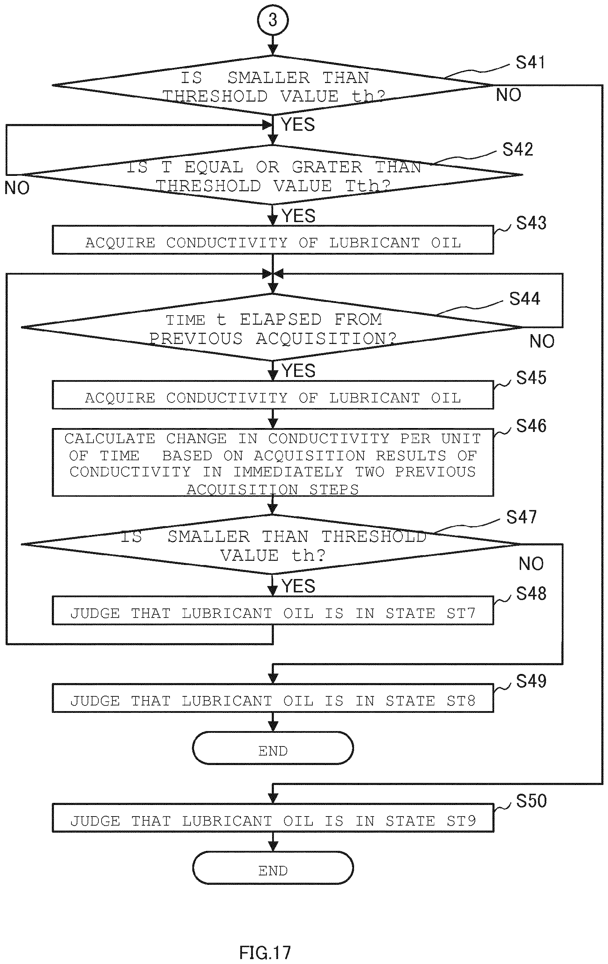

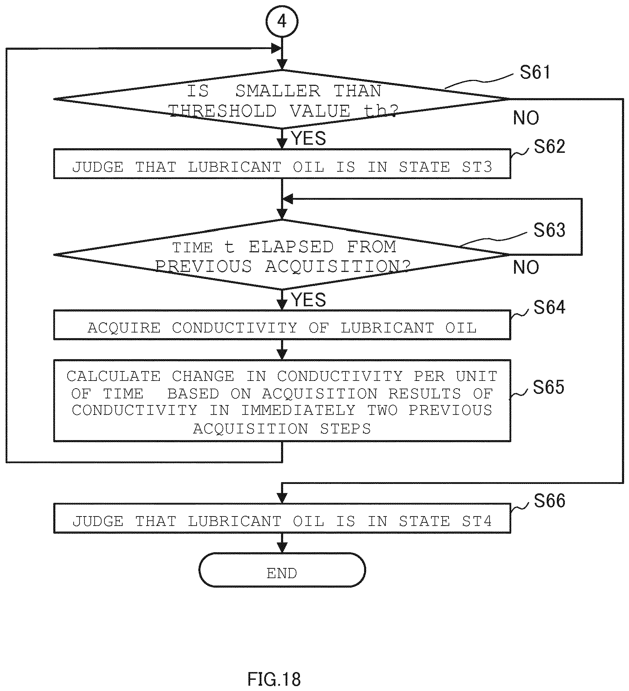

In Step S10, the sensor 1 instructs the state judgment unit 30 to judge whether or not the time T acquired in Step S5 is smaller than the threshold value Tth. When the state judgement unit 30 has judged that the time T is smaller than the threshold value Tth, the sensor 1 switches the operation to Step S41 shown in Fig. 17. Conversely, when the state judgement unit 30 has judged that the time T is equal to or greater than the threshold value Tth, the sensor 1 switches the operation to Step S61 shown in Fig. 18.

In Step S21 shown in Fig. 15, the sensor 1 instructs the state judgment unit 30 to judge whether or not the change in the dielectric constant per unit of time Δε is smaller than the threshold value Δεth. When the sensor 1 has judged by means of the state judgment unit 30 that the change in the dielectric constant per unit of time Δε is smaller than the threshold value Δεth, the sensor 1 switches the operation to Step S22. Conversely, when the sensor 1 has judged that the change in the dielectric constant per unit of time Δε is equal to or greater than the threshold value Δεth, the sensor 1 switches the operation to Step S26.

In Step S22, the sensor 1 judges by means of the state judgment unit 30 that the lubricant oil used in the device is in the state ST5. Subsequently, the sensor 1 switches the operation to Step S23.

In Step S23, the sensor 1 judges by means of the time measurement unit 20 whether or not a predetermined sampling time Δt or more has elapsed from the previous timing at which the characteristics acquisition 10 acquired the dielectric constant. When the sensor 1 has judged by means of the time measurement unit 20 that the elapsed time is equal to or greater than the sampling time Δt, the sensor 1 switches the operation to Step S24. Otherwise, the sensor 1 repeats the operation to Step S23.

In Step S24, the sensor 1 instructs the characteristics acquisition unit 10 to acquire the dielectric constant of the lubricant oil used in the device, following which the operation transits to Step S25.

In Step S25, the sensor 1 instructs the state judgment unit 30 to calculate the change in the dielectric constant per unit of time Δε based on the measurement values of the dielectric constant that were acquired by the characteristics acquisition unit 10 in two immediately previous executions of the acquisition step. Subsequently, the operation is returned to Step S21.

In Step S26, the sensor 1 judges by means of the state judgement unit 30 that the lubricant oil used in the device is in the state ST6, and the sensor 1 ends the operations shown in Figs. 14 through 18.

In Step S31 shown in Fig. 16, the sensor 1 instructs the state judgment unit 30 to judge whether or not the change in the dielectric constant per unit of time Δε is smaller than the threshold value Δεth. When the sensor 1 has judged by means of the state judgment unit 30 that the change in the dielectric constant per unit of time Δε is smaller than the threshold value Δεth, the sensor 1 switches the operation to Step S32. Conversely, when the sensor 1 has judged that the change in the dielectric constant per unit of time Δε is equal to or greater than the threshold value Δεth, the sensor 1 switches the operation to Step S36.

In Step S32, the sensor 1 judges by mean of the state judgment unit 30 that the lubricant oil used in the device is in the state ST2. Subsequently, the sensor 1 switches the operation to Step S33.

In Step S33, the sensor 1 judges by means of the time measurement unit 20 whether or not a predetermined sampling time Δt or more has elapsed from the previous timing at which the characteristics acquisition unit 10 acquired the dielectric constant. When the sensor 1 has judged by means of the time measurement unit 20 that the predetermined sampling time Δt or more has elapsed, the sensor 1 switches the operation to Step S34. Otherwise, the sensor 1 repeats the operation in Step S33.

In Step S34, the sensor 1 instructs the characteristics acquisition unit 10 to acquire the dielectric constant of the lubricant oil used in the device. Subsequently, the sensor 1 switches the operation to Step S35.

In Step S35, the sensor 1 instructs the state judgment unit 30 to calculate the change in the dielectric constant per unit of time Δε based on the measurement values of the dielectric constant that were acquired by the characteristics acquisition unit 10 in two immediately previous executions of the acquisition step. Subsequently, the sensor 1 returns the operation to Step S31.

In Step S36, the sensor 1 judges by mean of the state judgment unit 30 that the lubricant oil used in the device is in the state ST4, and the sensor 1 ends the operations shown in Figs. 14 through 18.

It should be noted that, as described above, the state ST4 can be classified into the three types shown in Figs. 6 through 8. In Step S36, the sensor 1 is capable of judging the state ST4 shown in Fig. 6, in addition to the state ST4 shown in Fig. 8. Description will be made below regarding a case in which judgment is made in Step S36 that the lubricant oil used in the device is in the state ST4 shown in Fig. 6.

In Step S31, the sensor 1 switches the operation according to whether or not the change in the dielectric constant per unit of time Δε is smaller than the threshold value Δεth. Accordingly, in actuality, such an arrangement has the potential to judge that the change in the dielectric constant per unit of time Δε has changed after the change in the conductivity per unit of time Δσ has changed, depending on the setting of the threshold value Δεth, even when the change in the dielectric constant per unit of time Δε and the change in the conductivity per unit of time Δσ have changed at the same timing as shown in Fig. 6. In this case, after judgment has been made in Step S32 that the lubricant oil is in the state ST2, the sensor 1 repeats the operations shown in Steps S33 through S35 and the operations shown in Steps S31 and S32 once or more. Subsequently, judgement is made in Step S36 that the state of the lubricant oil has transited from the state ST2 to the state ST4.

It should be noted that when the change in the dielectric constant per unit of time Δε and the change in the conductivity per unit of time Δσ have changed at the same timing as shown in Fig. 6, and when the sensor 1 has judged that the change in the dielectric constant per unit of time Δε and the change in the conductivity per unit of time Δσ have changed at the same timing, the operation is switched in Step S6 to Step S10 shown in Fig. 14. Accordingly, in this case, judgement is made in Step S66 shown in Fig. 18 that the lubricant oil is in the state ST4, detailed description of which will be made later with reference to Fig. 18.

In Step S41 shown in Fig. 17, the sensor 1 instructs the state judgment unit 30 to judge whether or not the change in the conductivity per unit of time Δσ is smaller than the threshold value Δσth. When the sensor 1 has judged by means of the state judgment unit 30 that the change in the conductivity per unit of time Δσ is smaller than the threshold value Δσth, the sensor 1 switches the operation to Step S42. Conversely, when the sensor 1 has judged that the change in the conductivity per unit of time Δσ is equal to or greater than the threshold value Δσth, the sensor 1 switches the operation to Step S50.

In Step S42, the sensor 1 instructs the state judgment unit 30 to judge whether or not the time acquired in Step S5 is smaller than the threshold value Tth. When the state judgement unit 30 has judged that the time is equal to or greater than the threshold value Tth, the sensor 1 switches the operation to Step S43. Conversely, when the state judgement unit 30 has judged that the time is smaller than the threshold value Tth, the sensor 1 repeats the operation in Step S42.

In Step S43, the sensor 1 instructs the characteristics acquisition unit 10 to acquire the conductivity of the lubricant oil used in the device, following which the operation transits to Step S44.

In Step S44, the sensor 1 judges by means of the time measurement unit 20 whether or not a predetermined sampling time Δt or more has elapsed from the previous timing at which the characteristics acquisition 10 acquired the conductivity. When the sensor 1 has judged by means of the time measurement unit 20 that the predetermined sampling time Δt or more has elapsed, the sensor 1 switches the operation to Step S45. Conversely, When the sensor 1 has judged by means of the time measurement unit 20 that the predetermined sampling time Δt or more has not elapsed, the sensor 1 repeats the operation in Step S44.

In Step S45, the sensor 1 instructs the characteristics acquisition unit 10 to acquire the conductivity of the lubricant oil used in the device. Subsequently, the sensor 1 switches the operation to Step S46.

In Step S46, the sensor 1 instructs the state judgment unit 30 to calculate the change in the conductivity per unit of time Δσ based on the measurement values of the conductivity that were acquired by the characteristics acquisition unit 10 in two immediately previous executions of the acquisition step. Subsequently, the sensor 1 switches the operation to Step S47.

In Step S47, the sensor 1 instructs the state judgment unit 30 to judge whether or not the change in the conductivity per unit of time Δσ is smaller than the threshold value Δσth. When the sensor 1 has judged by means of the state judgment unit 30 that the change in the conductivity per unit of time Δσ is smaller than the threshold value Δσth, the sensor 1 switches the operation to Step S48. Conversely, when the sensor 1 has judged that the change in the conductivity per unit of time Δσ is equal to or greater than the threshold value Δσth, the sensor 1 switches the operation to Step S49.

In Step S48, the sensor 1 judges by mean of the state judgment unit 30 that the lubricant oil used in the device is in the state ST7. Subsequently, the sensor 1 returns the operation to Step S44.

In Step S49, the sensor 1 judges by mean of the state judgment unit 30 that the lubricant oil used in the device is in the state ST8. Subsequently, the sensor 1 ends the operations shown in Figs. 14 through 18.

In Step S50, the sensor 1 judges by mean of the state judgment unit 30 that the lubricant oil used in the device is in the state ST9. Subsequently, the sensor 1 ends the operations shown in Figs. 14 through 18.

In Step S61 shown in Fig. 18, the sensor 1 instructs the state judgment unit 30 to judge whether or not the change in the conductivity per unit of time Δσ is smaller than the threshold value Δσth. When the sensor 1 has judged by means of the state judgment unit 30 that the change in the conductivity per unit of time Δσ is smaller than the threshold value Δσth, the sensor 1 switches the operation to Step S62. Conversely, when the sensor 1 has judged that the change in the conductivity per unit of time Δσ is equal to or greater than the threshold value Δσth, the sensor 1 switches the operation to Step S66.

In Step S62, the sensor 1 judges by mean of the state judgment unit 30 that the lubricant oil used in the device is in the state ST3. Subsequently, the sensor 1 switches the operation to Step S63.

In Step S63, the sensor 1 judges by means of the time measurement unit 20 whether or not a predetermined sampling time Δt or more has elapsed from the previous timing at which the characteristics acquisition unit 10 acquired the conductivity. When the sensor 1 has judged by means of the time measurement unit 20 that the predetermined sampling time Δt or more has elapsed, the sensor 1 switches the operation to Step S64. Otherwise, the sensor 1 repeats the operation in Step S63.

In Step S64, the sensor 1 instructs the characteristics acquisition unit 10 to acquire the conductivity of the lubricant oil used in the device. Subsequently, the sensor 1 switches the operation to Step S65.

In Step S65, the sensor 1 instructs the state judgment unit 30 to calculate the change in the conductivity per unit of time Δσ based on the measurement values of the conductivity that were acquired by the characteristics acquisition unit 10 in two immediately previous executions of the acquisition step. Subsequently, the sensor 1 returns the operation to Step S61.

In Step S66, the sensor 1 judges by mean of the state judgment unit 30 that the lubricant oil used in the device is in the state ST4. Subsequently, the sensor 1 ends the operations shown in Figs. 14 through 18.

It should be noted that, as described above, the state ST4 can be classified into the three types shown in Figs. 6 through 8. In Step S66, the sensor 1 is capable of judging the state ST4 shown in Fig. 6, in addition to the state ST4 shown in Fig. 7. Description will be made below regarding a case in which judgment is made in Step S66 that the lubricant oil used in the device is in the state ST4 shown in Fig. 6.

In Step S61, the sensor 1 switches the operation according to whether or not the change in the conductivity per unit of time Δσ is smaller than the threshold value Δσth. Accordingly, in actuality, such an arrangement has the potential to judge that the change in the conductivity per unit of time Δσ has changed after the change in the dielectric constant per unit of time Δε has changed, depending on the setting of the threshold value Δσth, even when the change in the dielectric constant per unit of time Δε and the change in the conductivity per unit of time Δσ have changed at the same timing as shown in Fig. 6. In this case, after judgment has been made in Step S62 that the lubricant oil is in the state ST3, the sensor 1 repeats the operations shown in Steps S63 through S65 and the operations shown in Steps S61 and S62 once or more. Subsequently, judgement is made in Step S66 that the state of the lubricant oil has transited from the state ST3 to the state ST4.

On the other hand, when the change in the dielectric constant per unit of time Δε and the change in the conductivity per unit of time Δσ have changed at the same timing as shown in Fig. 6, and when the sensor 1 has judged that the change in the dielectric constant per unit of time Δε and the change in the conductivity per unit of time Δσ have changed at the same timing, judgment is made in Step S66 that the lubricant oil is in the state ST4 without involving such a judgment step in which the lubricant oil is in the state ST3.

The sensor 1 instructs the characteristics acquisition unit 10 to acquire the dielectric constant and the conductivity of the lubricant oil, and instructs the time measurement unit 20 to measure the time from the time point at which the lubricant oil was charged to the device. Furthermore, the sensor 1 instructs the state judgment unit 30 to judge the state of the lubricant oil based on the dielectric constant and the conductivity acquired by the characteristics acquisition unit 10 and the time measured by the time measurement unit 20. This means that the sensor 1 judges the state of the lubricant oil based on a combination of the dielectric constant and the conductivity of the lubricant oil and the time that has elapsed from the time point at which the lubricant oil was charged to the device. This provides further detailed judgment with respect to the state of the lubricant oil.

In a case in which an abnormal state has occurred in the lubricant oil, a large change occurs in at least one of the dielectric constant and the conductivity of the lubricant oil. Accordingly, in a case in which an abnormal state has occurred in the lubricant oil, a large change occurs in at least one of the change in the dielectric constant per unit of time Δε and the change in the conductivity per unit of time Δσ.

Thus, the sensor 1 instructs the state judgment unit 30 to calculate the change in the dielectric constant per unit of time Δε based on the measurement values of the dielectric constant acquired by the characteristics acquisition unit 10. Furthermore, the sensor 1 instructs the state judgment unit 30 to calculate the change in the conductivity per unit of time Δσ based on the measurement values of the conductivity acquired by the characteristics acquisition unit 10. Moreover, the sensor 1 instructs the state judgment unit 30 to judge the state of the lubricant oil based on the change in the dielectric constant per unit of time Δε and the change in the conductivity per unit of time Δσ thus acquired, and the time T measured by the time measurement unit 20 at the time point at which the change in the dielectric constant per unit of time Δε and the change in the conductivity per unit of time Δσ were acquired. This allows judgment to be appropriately made with respect to the timing at which an abnormal state has occurred in the lubricant oil. This provides further detailed judgment with respect to the state of the lubricant oil.

Furthermore, the sensor 1 instructs the state judgement unit 30 to acquire the timing at which the change in the dielectric constant per unit of time Δε becomes equal to or larger than the threshold value Δεth, and the timing at which the change in the conductivity per unit of time Δσ becomes equal to or larger than the threshold value Δσth. Subsequently, the state judgment unit 30 judges the state of the lubricant oil based on the two timings thus acquired. This provides appropriate judgment with respect to the timing at which an abnormal state has occurred in the lubricant oil. Thus, this provides further detailed judgment with respect to the state of the lubricant oil.

It should be noted that description will be made in the present embodiment regarding an arrangement in which measurement is performed twice for each of the dielectric constant and the conductivity. Furthermore, the change in the dielectric constant per unit of time Δε and the change in the conductivity per unit of time Δσ are calculated based on the measurement results thus acquired. However, the present invention is not restricted to such an arrangement. Also, measurement may be performed three times or more for each of the dielectric constant and the conductivity. In this case, assuming a graph with a vertical axis that represents the dielectric constant and a horizontal axis that represents time, the slope of a regression line may be calculated for the measurement points of the dielectric constant acquired by the characteristics acquisition unit 10, and the slope thus calculated may be employed as the change in the dielectric constant per unit of time Δε. Also, in the same way as in the calculation of the change in the dielectric constant per unit of time Δε, the slope of a regression line may be calculated for the measurement points of the conductivity acquired by the characteristics acquisition unit 10, and the slope thus calculated may be employed as the change in the conductivity per unit of time Δσ.

Description has been made in the present embodiment regarding an arrangement in which the sensor 1 detects the state of the lubricant oil. However, the present invention is not restricted to such an arrangement. Also, the sensor 1 is able to provide fluid state detection regardless of the kind of fluid. For example, this arrangement is capable of detecting the contamination level of water, the quality of a chemical or drinking water, etc.

Description has been made in the present embodiment regarding an arrangement in which the planar-shaped electrodes 12 and 13 are arranged in parallel. However, the present invention is not restricted to such an arrangement. For example, the electrodes 12 and 13 may be formed in the form of a cylindrical shape or a cylindroid shape. Also, the electrodes 12 and 13 may be respectively formed in two cylindrical shapes having different inner diameters. Also, one of the electrodes 12 and 13 may be arranged in the internal space of the other with the central axes thereof aligned, as with a coaxial cylindrical capacitor, for example.

Description has been made in the present embodiment regarding an arrangement in which the sensor 1 repeatedly executes the operations shown in Figs. 14 through 18 starting from Step S1 every time a predetermined period of time elapses, so as to repeatedly acquire the change in the dielectric constant per unit of time Δε and the change in the conductivity per unit of time Δσ. Furthermore, the state of the lubricant oil used in the device is judged based on the change in the dielectric constant per unit of time Δε, the change in the conductivity per unit of time Δσ, the time period from the timing at which the lubricant oil was charged to the interior of the device up to the timing at which the change in the dielectric constant per unit of time Δε was acquired, and the time period from the timing at which the lubricant oil was charged to the interior of the device up to the timing at which the change in the conductivity per unit of time Δσ was acquired.

However, the present invention is not restricted to such an arrangement. Also, the change in the dielectric constant per unit of time Δε and the change in the conductivity per unit of time Δσ may be acquired at a timing after a time period T1 has elapsed from the charging of the lubricant oil to the device and at a timing after a time period T2 has elapsed from the charging of the lubricant oil to the device. Furthermore, the state of the lubricant oil may be judged based on the change in the dielectric constant per unit of time Δε acquired at the timing after the time period T1 has elapsed from the charging of the lubricant oil to the device, the change in the dielectric constant per unit of time Δε acquired at the timing after the time period T2 has elapsed from the charging of the lubricant oil to the device, the change in the conductivity per unit of time Δσ at the timing after the time period T1 has elapsed from the charging of the lubricant oil to the device, and the change in the conductivity per unit of time Δσ at the timing after the time period T2 has elapsed from the charging of the lubricant oil to the device. It should be noted that the time T1 is a time period that is equal to or smaller than the threshold value Tth, and may be designed as appropriate according to the kind of the lubricant oil, the kind of the device, the operation state of the device, etc. For example, the time T1 may be set to a time period on the order of several ten hours. Also, the time T2 is a time period that is equal to or larger than the threshold value Tth, and may be designed as appropriate according to the kind of the lubricant oil, the kind of the device, the operation state of the device, etc. For example, the time T2 may be set to a time period on the order of several hundred hours.

It should be noted that the periods of time T1 and T2 may be designed according to a bathtub curve. For example, in a case in which the recommended time after which the lubricant oil charged to the device is to be replaced is one year or 3,000 hours, the time T2 is designed to be shorter than the recommended lubricant oil replacement time.

Also, the change in the dielectric constant per unit of time Δε and the change in the conductivity per unit of time Δσ may be acquired once or more in a period from the time point at which the time T1 has elapsed from the charging of the lubricant oil to the device up to the time point at which the time T2 has elapsed from the charging of the lubricant oil to the device. Also, the change in the dielectric constant per unit of time Δε and the change in the conductivity per unit of time Δσ may be acquired once or more in a period of the time T2 that elapsed from the charging of the lubricant oil to the device. It should be noted that the probability of the occurrence of a malfunction in the sensor 1 is relatively low in the period from the time point at which the time T1 has elapsed from the charging of the lubricant oil to the device up to the time point at which the time T2 has elapsed from the charging of the lubricant oil to the device. Accordingly, it is not important to acquire the change in the dielectric constant per unit of time Δε and the change in the conductivity per unit of time Δσ in this period.

Also, in the present embodiment, immediately before Step S26 shown in Fig. 15, the sensor 1 may execute a step in which the time measurement unit 20 acquires the time T that has elapsed from the timing at which the lubricant oil was charged to the device and a step in which the state judgement unit 30 judges whether or not the time T thus acquired is smaller than the threshold value Tth. In this case, when the sensor 1 has judged by means of the state judgment unit 30 that the time T is equal to or larger than the threshold value Tth, the sensor 1 may switch the operation to Step S26. Conversely, when the sensor 1 has judged that the time T is smaller than the threshold value Tth, the sensor 1 may switch the operation to Step S50 shown in Fig. 17. With such an arrangement, when the change in the dielectric constant per unit of time Δε and the change in the conductivity per unit of time Δσ have changed before the timing at which the time of the threshold value Tth has elapsed from the charging of the lubricant oil to the device, the sensor 1 judges that the lubricant oil used in the device is in the state ST9 even when the change in the dielectric constant per unit of time Δε and the change in the conductivity per unit of time Δσ have changed at different timings.

Description has been made above regarding the present embodiment. With this embodiment, this provides further detailed judgment with respect to the state of a fluid.

It should be noted that although detailed description has been made regarding the embodiment of the present invention with reference to the drawings, specific configurations thereof are not restricted to this embodiment. Rather, various changes of design may be made, which are encompassed by the present invention without departing from the spirit or scope of the appended claims.

Also, the present invention may be applicable to a hydraulic device, for example.

### Reference Signs List

1 sensor
10 characteristics acquisition unit
11 base
12, 13 electrode
14 power supply unit
15 acquisition unit
20 time measurement unit
30 state judgment unit

## Claims

1. A sensor that judges a state of a fluid in a device, comprising:
a characteristics acquisition unit that acquires a dielectric constant and a conductivity of the fluid;
a time measurement unit that measures a time period from a timing at which the fluid was charged to the device; and
a state judgment unit that judges the state of the fluid based on the dielectric constant and the conductivity acquired by the characteristics acquisition unit and the time period measured by the time measurement unit.

2. The sensor according to claim 1, wherein the state judgment unit acquires a change in the dielectric constant per unit of time based on the dielectric constant acquired by the characteristics acquisition unit,
wherein the state judgment unit acquires a change in the conductivity per unit of time based on the conductivity acquired by the characteristics acquisition unit,
and wherein the state judgment unit judges the state of the fluid based on the change in the dielectric constant per unit of time thus acquired, the change in the conductivity per unit of time thus acquired, the time period measured by the time measurement unit at a timing at which the change in the dielectric constant per unit of time was acquired, and the time period measured by the time measurement unit at a timing at which the change in the conductivity per unit of time was acquired.

3. The sensor according to claim 2, wherein the state judgment unit acquires a timing at which the change in the dielectric constant per unit of time becomes equal to or larger than a dielectric constant threshold value based on the change in the dielectric constant per unit of time thus acquired and the time period measured by the time measurement unit at the time at which the change in the dielectric constant per unit of time was acquired,
wherein the state judgment unit acquires a timing at which the change in the conductivity per unit of time becomes equal to or larger than a conductivity threshold value based on the change in the conductivity per unit of time thus acquired and the time period measured by the time measurement unit at the time at which the change in the conductivity per unit of time was acquired,
and wherein the state judgment unit judges the state of the fluid based on the aforementioned two timings.

4. The sensor according to claim 1, wherein the state judgment unit acquires the change in the dielectric constant per unit of time at a timing at which a predetermined first time period has been measured by the time measurement unit and at a second timing at which a predetermined second time period that is longer than the first time period has been measured by the time measurement unit, based on results acquired for the dielectric constant acquired by the characteristics acquisition unit,
wherein the state judgment unit acquires the change in the conductivity per unit of time at a timing at which the predetermined first time period has been measured by the time measurement unit and at a timing at which the predetermined second time period has been measured by the time measurement unit, based on results acquired for the conductivity acquired by the characteristic acquisition unit,
and wherein the state judgment unit judges the state of the fluid based on the change in the dielectric constant per unit of time acquired at the timing at which the first time period has been measured by the time measurement unit, the change in the dielectric constant per unit of time acquired at the timing at which the second time period has been measured by the time measurement unit, the change in the conductivity per unit of time acquired at the timing at which the first time period has been measured by the time measurement unit, and the change in the conductivity per unit of time acquired at the timing at which the second time period has been measured by the time measurement unit.
